# EUROPEAN PATENT APPLICATION

(11) **EP 2 759 305 A1**
(43) Date of publication of application: **30.07.2014**
(21) Application number: 13152475.3
(22) Date of filing: 24.01.2013
(51) Int. Cl.: A61K 38/48, A61K 38/57

(54) **Hemostatic compositions**

(71) Applicant: Thrombotargets Europe, S.L., 08860 Castelldefels - Barcelona (ES)
(72) Inventor: Rodríguez Fernández - Alba, Juan Ramon., 08860 CASTELLDEFELS (ES); Murat Moreno, Jesus., 08860 CASTELLDEFELS (ES)
(74) Representative: ZBM Patents - Zea, Barlocci & Markvardsen

(57) **Abstract**

The invention provides a composition comprising a combination of lipidated tissue factor and thrombin, wherein the amount of thrombin is comprised from 1 to 30 IU per gram of composition, and the amount of lipidated tissue factor is comprised from 5 to 150 ng per gram of composition.

The present invention also provides pharmaceutical or veterinary compositions as well as hemostatic compositions.

The compositions of the invention are useful in the treatment of hemorrhages.

## Description

The present invention is in the field of hemostatic compositions.

### BACKGROUND ART

The set of physiological mechanisms triggered to stop bleeding after an injury is known as hemostasis. Hemostasis is a very complex process that involves a variety of molecules and cell types. To facilitate its study, hemostasis can be subdivided into four overlapping steps: i) vasoconstriction, ii) formation in the area of the wound of an initial platelet plug, iii) formation of a fibrin resistant clot (clotting cascade), and iv) a clot dissolution step or fibrinolysis, from which the healing process of the wound starts.

The coagulation cascade is initiated upon interaction of Factor VII (FVII), in its soluble and inactive form in plasma, with its specific receptor known as tissue factor (hereinafter also referred as "TF"). In its native form, TF is located predominantly on the surface of various cell types surrounding blood vessels. Therefore, under normal conditions, it contacts uniquely with plasmatic molecules in the case of an injury or wound. After the rupture of a blood vessel, the coagulation cascade is initiated by the association of TF with FVII, triggering the subsequent interaction between the various blood coagulation factors. The end result is the formation of thrombin (FIIa) which, in turn, converts the fibrinogen (FI), which is very abundant in plasma, into fibrin fibers. These fibrin fibers are insoluble, and form a mesh, which, together with platelets, results in what it is known as a fibrin clot.

With the aim of facilitating a rapid hemostasis in cases of massive hemorrhage, various procedures have been used over centuries, such as direct pressure, packing with gauze, suture-like threads, and tourniquet. These techniques are known as "non-active" hemostatic means, and assist in the hemostatic process when used with different types of dressings. Nowadays, the most used dressings are based on biocompatible and absorbable materials such as collagen fibers, gelatins or oxidized cellulose fibers.

In an attempt to improve surgical hemostatic settings, during the past decades a strong effort has been done in developing new products containing "active" ingredients which, in addition to traditional techniques, could help in the improvement of hemostasis. In this regard, there are drugs available in the market ("active hemostatic means") either containing the whole set of coagulation factors in plasma concentrates or based on isolates factors with a high degree of purity.

Among them, coagulation Factor VII (FVII) , coagulation factor VIII (FVIII), coagulation factor IX (FIX), and coagulation factor XI (FXI) are already marketed. They should be used by intravenous route to overcome various plasma deficiencies responsible for certain diseases such as hemophilia. Nevertheless, only thrombin, also known as activated Factor II (FIIa), or compositions comprising thereof, is used as topical hemostatic agent to be applied on skin lesions or over bleeding wounds in surgery. In fact, from the 50s, FIIa has been widely used in multiple surgical applications. In many of the disclosed applications, FIIa is associated to a support of sponge-type, gauze, or gel, such as collagen gel or gelatins

FIIa acts at the latest steps of the coagulation cascade, directly on the fibrinogen (FI), facilitating a rapid clot formation. Therefore, its efficacy does not require the activation of the remaining clotting factors. However, to ensure its efficiency, the FIIa has to be applied on bleeding surfaces at high concentrations. This is because there are many factors reducing their active concentration, such as: i) its concentration is diluted due to blood flow, ii) there is a rapid inactivation due to its interaction with its natural inhibitor, antithrombin III, iii) there is an additional inactivation due to its immobilization in thrombus formation, and iv) its mechanical removal by gauzes or by irrigation of the surgical procedure. Such factors (i)-(iv) obligates the use of therapeutically FIIa concentrations higher than those required during the physiological clot formation. In certain surgical activities, wherein the use of heparin is recommended, the recommended FIIa concentration is increased.

Despite its usefulness, there have been disclosed many side-effects due to the use of such high amounts of FIIa. Among them: (a) as being a pro-inflammatory molecule, such concentrations can give rise to an non-physiological inflammation process; (b) there is an immunogenicity risk because such thrombin is, in many cases, obtained from non-human -animal plasma, and (c) FIIa obtained from plasma from human origin has the risk of containing adventitious agents. In addition to (a)-(c), and independently of the concentration used, thrombin by itself is not effective in inducing an appropriate blood clotting propagation, a requirement for a fast hemostasis.

Therefore, in view of the above, there is the need of further hemostatic compositions with an appropriate hemostatic profile.

### SUMMARY OF THE INVENTION

The present inventors have found that adding lipidated tissue factor (TF) to thrombin, the hemostatic effect of thrombin is substantially increased, in such a way that the amount of thrombin needed to get the desired effect can be reduced.

In particular, when a composition comprising a combination of lipidated tissue factor and thrombin was prepared, it was found that the hemostatic effect of thrombin was synergistically increased in such a way that it was possible to use small amounts of thrombin (from 1 to 30 IU per gram of composition) when thelipidated TF was present in the composition at a concentration of 5-150 ng per gram of composition.

As it is summarized in Table 2 below, for instance, using a composition as the one of the invention, comprising 1 IU of thrombin/gram of composition, the clotting time was reduced about at least 5 times when lipidated TF was incorporated at a concentration of 5, 15, or 150 ng/gram of composition. Substantial reductions in the clotting time were also observed when compositions comprising 3 UI or 30 UI of thrombin/gram composition were made together with lipidated TF at concentrations of 5, 15, and 150 ng/gram of composition.

Therefore, these experimental data allows concluding that when thrombin is combined with lipidated TF, a great reduction in the time needed to initiate the fibrin network formation is observed. This means that administering lipidated TF together with thrombin, a very strong hemostatic effect is obtained.

Thus, in a first aspect the present invention provides a composition comprising a combination of lipidated tissue factor and thrombin, wherein the amount of thrombin is comprised from 1 to 30 IU per gram of composition, and the amount of lipidated tissue factor is comprised from 5 to 150 ng per gram of composition.

In addition to the above, the compositions of the invention, including low concentrations of thrombin, provides a hemostatic effect which can be substantially the same than the one provided by the administration of 300 UI of thrombin/mL.

As it has been stated above, administering the composition of the invention, using low amounts of thrombin much less time is required to stop bleeding.

This is of special relevance in cases of internal traumatisms, wherein compressions with thrombin or other hemostatic products are not efficient enough to stop bleeding, being a high risk that the patient dies. With the composition of the invention the coagulation occurs much faster, which means a great advance in cases of internal traumatisms wherein the time for stop bleeding is critical for patient's survival.

In addition, using low amounts of thrombin, the composition of the invention avoids the problems associated to the administration of high amounts of thrombin disclosed in the prior art, such as inflammation and side-immunogenic effects, among others.

The composition of the first aspect of the invention can include other ingredients, such as fibrinolysis inhibitors, CaCl₂, etc. provided that said other ingredients do not negatively affect to the hemostatic activity of the combination of thrombin and lipidated tissue factor.

Due to the properties of the composition of the first aspect of the invention, it can be used in the form of pharmaceutical or veterinary compositions.

Therefore, in a second aspect the present invention provides a pharmaceutical or veterinary composition comprising a therapeutically effective amount of the composition of the first aspect of the invention, together with other appropriate pharmaceutically or veterinary acceptable excipients and/or carriers.

Due to the excellent hemostatic properties of the composition of the first aspect of the invention (Table 2 below), both the composition of the first aspect of the invention as well as the pharmaceutical or veterinary composition of the second aspect of the invention can be used in the formulation of a hemostatic composition together with an appropriate carrier material.

Thus, in a third aspect the present invention provides a hemostatic composition comprising the composition as defined in the first aspect of the invention, or the pharmaceutical or veterinary composition as defined in the second aspect of the invention, and a carrier material.

In a fourth aspect the present invention provides the use of the composition of the first aspect of the invention or of the pharmaceutical or veterinary composition of the second aspect of the invention as a hemostatic agent.

As it has been discussed above, compositions of the invention, due to its hemostatic effect, are useful in the treatment of hemorrhages.

Thus, in a fifth aspect the present invention provides the composition of the first aspect of the invention or the pharmaceutical or veterinary composition of the second aspect of the invention or the hemostatic composition of the third aspect of the invention, for use as a medicine. This aspect can be formulated as a method for the treatment of a disease comprising administering to a subject in need thereof a therapeutically effective amount of the composition of the first aspect of the invention or of the pharmaceutical or veterinary composition of the second aspect of the invention or of the hemostatic composition of the third aspect of the invention together with other appropriate pharmaceutically or veterinary acceptable excipients and/or carriers.

In a sixth aspect the present invention provides the composition of the first aspect of the invention or the pharmaceutical or veterinary composition of the second aspect of the invention or the hemostatic composition of the third aspect of the invention, for use in the treatment of haemorrhages.

This aspect can alternatively be formulated as the use of the composition of the first aspect of the invention or of the pharmaceutical or veterinary composition of the second aspect of the invention or of the hemostatic composition of the third aspect of the invention in the manufacture of a medicament for the treatment of hemorrhages. This aspect can be alternatively formulated as a method for the treatment of hemorrhages, the method comprising administering to a subject in need thereof a therapeutically effective amount of the composition of the first aspect of the invention or of the pharmaceutical or veterinary composition of the second aspect of the invention or of the hemostatic composition of the third aspect of the invention together with other appropriate pharmaceutically or veterinary acceptable excipients and/or carriers.

In a further aspect the present invention provides the use of the composition of the first aspect of the invention or of the pharmaceutical or veterinary composition of the second aspect of the invention or of the hemostatic composition of the third aspect of the invention as wound healing agent.

Finally, the compositions of the invention can be provided as a kit.

Thus, in an eighth aspect the present invention provides a kit comprising the composition as defined in the first aspect of the invention, or the pharmaceutical composition as defined in the second aspect of the invention, and an applicator that allows the simultaneous application of both thrombin, and lipidated tissue factor.

### DETAILED DESCRIPTION OF THE INVENTION

As it has been stated above, in a first aspect the present invention provides a composition comprising thrombin and lipidated tissue factor.

In one embodiment, the composition of the first aspect of the invention is in solid form, semi-solid form, or liquid form.

In the present invention the term "solid form" is to be understood as including powder, and solid foams.

In the present invention, the term "semi-solid form" is to be understood as including ointments, pastes, creams, soft or hard gels, suspensions, emulsions, and suppositories.

In the present invention, the term "liquid form" is to be understood as including liquid foams, solutions, and lotions.

In one embodiment, the composition of the first aspect of the invention comprises thrombin at a concentration comprised from 10 to 30 IU per gram of composition.

In another embodiment, the composition of the first aspect of the invention comprises thrombin at a concentration of 1 IU per gram of composition, 3 IU per gram of composition or 30 IU per gram of composition.

In another embodiment, the composition of the first aspect of the invention comprises lipidated tissue factor at a concentration comprised from 5 to 15 ng per gram of composition.

In another embodiment, the composition of the first aspect of the invention comprises lipidated tissue factor at a concentration of 150 ng per gram of composition.

In another embodiment, the composition of the first aspect of the invention is one selected from the group consisting of:
- 1 IU of thrombin per gram of composition and 5 ng of lipidated TF per gram of composition;
- 1 IU of thrombin per gram of composition and 15 ng of lipidated TF per gram of composition;
- 1 IU of thrombin per gram of composition and 150 ng of lipidated TF per gram of composition;
- 3 IU of thrombin per gram of composition and 5 ng of lipidated TF per gram of composition;
- 3 IU of thrombin per gram of composition and 15 ng of lipidated TF per gram of composition;
- 3 IU of thrombin per gram of composition and 150 ng of lipidated TF per gram of composition;
- 30 IU of thrombin per gram of composition and 5 ng of lipidated TF per gram of composition;
- 30 IU of thrombin per gram of composition and 15 ng of lipidated TF per gram of composition; and
- 30 IU of thrombin per gram of composition and 150 ng of lipidated TF per gram of composition.

In another embodiment, the composition of the first aspect of the invention is one selected from the group consisting of:
- 1 IU of thrombin per gram of composition and 150 ng of lipidated TF per gram of composition;
- 3 IU of thrombin per gram of composition and 150 ng of lipidated TF per gram of composition; and
- 30 IU of thrombin per gram of composition and 150 ng of lipidated TF per gram of composition.

In the present invention, the term "tissue factor" (TF) has to be understood as an integral membrane glycoprotein that is widely distributed in the animal kingdom. TF appears in different cells types, predominantly in the subendothelial tissue, and is necessary for the initiation of the extrinsic pathway of coagulation cascade leading to the production of FIIa and the formation of a stable fibrin clot. Exemplary TF proteins that can be used in the present invention include human TF (UniProtKB/Swiss-Prot. Version 148, November 2012, accession number P13726), mouse TF (UniProtKB/SwissProt. Version 103, November 2012, accession number P20352), bovine TF (UniProtKB/Swiss-Prot. Version 89, November 2012, accession number P30931), rabbit TF (UniProtKB/Swiss-Prot. Version 90, November 2012, accession number P24055) and TF proteins of different animals.

The human TF protein with the SwissProt accession number P13726 has a well-defined domain structure which comprises (1) a signal peptide or a region with a 32 amino acid leader sequence that is post-translationally processed from the immature to the mature form; (2) an N-glycosylated hydrophilic extracellular domain comprising 219 amino acids; (3) a highly hydrophobic fragment of 23 amino acids, which form the transmembrane domain; and (4) the 21-amino acid carboxyl end which is the protein cytoplasmic fragment.

In a particular embodiment, the TF corresponds to the mature TF.

The term "mature TF" as used herein, refers to the TF protein whose amino acid sequence lacks the signal peptide. In an embodiment, said mature TF protein is the mature human TF of SEQ ID NO: 1.

In one embodiment, the TF is glycosylated.

As used herein, the term "glycosylated" includes any degree of glycosylation. Since native TF contains several glycosylation sites, as explained in detail below, the expression "tissue factor" also encompasses those forms of the protein showing different degrees of glycosylation, said forms being obtained by expressing TF in hosts capable of carrying out N-glycosylation reactions. In addition, the term "tissue factor" also embraces those variants resulting from the insertion, deletion, or substitution of one or more amino acid residues in the native TF sequence. Suitable functional assays that can be used to assess whether a given polypeptide is a functional variant of TF are those assays based on the determination of the ability of the TF variant to specifically bind FVIIa, or on the *in vitro* determination of the coagulation time in plasma or whole blood, by an *in vivo* assay in a severe hemorrhage animal model or by an *in vivo* assay in a lethal hemorrhage animal model. Procedures for carrying out these assays have been disclosed in the prior art.

Such variants according to the present invention include amino acid sequences that are at least 60%, 70%, 80%, 90%, 95% or 96% identical to the native TF molecules mentioned above. As known in the art the "identity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm.

The TF may be fully glycosylated, partially glycosylated or non-glycosylated.

In the present application "fully glycosylated" means that all potential glycosylation sites have been glycosylated.

In the present application "partially glycosilated" means that at least one of the three N-glycosylation sites is non-functional, being not possible its glycosilation.

In the present application "non-glycosylated" means that none of the N-glycosylation sites is functional, or alternatively, that the TF polypeptide has been expressed by a non-glycosilation method in vitro (i.e. cell-free expression system), or in a vector deprived of metabolic glycosylation pathways (i.e. *E*. *coli*).

In an embodiment, the TF is partially glycosylated or non-glycosylated. In this embodiment, the TF has been modified in such a way that at least one of the N-glycosylation sites is non-functional, being not possible its glycosylation.

All mature TF proteins contain three potential N-linked glycosylation sites having the consensus sequence Asn-Xaa-Ser/Thr. In the case of human mature TF, such three glycosilation sites are located at Asn11 (sequence Asn11-Leu12-Thr13), Asn124 (sequence Asn124-Val125-Thr126) and Asn137 (sequence Asn137-Asn138-Thr139), said positions being in respect of SEQ ID NO: 1.

In an embodiment, the N-glycosylation site or sites which can be modified to make them non-functional in the human mature TF protein are those corresponding to the N-glycosylation sites NLT at positions 11-13, NVT at positions 124-126, and NNT at positions 137-139 in the mature human TF of sequence SEQ ID NO: 1 above.

In another embodiment, the TF carries one or more substitutions of the Asn residues into residues which are not acceptors for N-glycosylation. In a still more preferred embodiment, the TF variant comprises one or more Asn-to-Ala mutations in the Asn residues in positions corresponding to positions 11, 124 or 137 in the mature human TF. Preferably, the mature human TF carries the mutation Asn to Ala at position 124 of SEQ ID NO: 1.

The glycosylation will vary depending on the expression system used for the production of TF. For instance, the tissue factor protein can include one or more plant-specific glycan, yeast-specific glycan, insect-specific glycan, or animal-specific glycan.

When TF is produced in yeast, the glycosylation typically will involve an inner core of about ten mannose residues, linked to the asparagine via two GlcNAc residues, and a branched outer chain of 50-100 mannose residues. Therefore the N-linked glycosylation could potentially add as many as 300 mannose residues to TF, an increase in molecular mass in about 60 kDa. Furthermore, it is also possible that several mannose residues could be attached to various O-linked glycosylation sites (more than 25). Therefore, TF molecules included in the compositions of the present invention have a variable degree and composition of N-linked glycosilation in one or more N-glycosylation sites. In still another embodiment, the TF protein is a fusion protein, said fusion protein comprising a first portion, which comprises said TF protein, and a second portion, which comprises another peptide or protein.

Said second portion may be bound to the N-terminus first portion of said TF protein fragment, or, alternatively said second portion may be bound to the C-terminus region of said TF protein fragment. Both first and second portions may be directly bound or bound through a linker polypeptide between said first and second regions.

In another embodiment, said second portion is a tag. The tag can be bound to the C-terminal or N- terminal domain of the TF protein first portion.
In the present invention, the term "tag" has to be understood as any peptide or amino acid sequence, bound to the C-terminal or N-terminal domain of said TF protein, which can be used in the isolation or purification of the fusion protein. Thus, said tag is capable of binding to one or more ligands, such as, for example, one or more ligands of an affinity matrix such as a chromatography support or bead with high affinity. An example of said tag is a histidine tag (His-tag or HT), such as a tag comprising 6 residues of histidine (His6 or H6), which can bind to a column of nickel (Ni²⁺) or cobalt (Co²⁺) with high affinity. Additional illustrative, non-limitative, examples of tags useful for isolating or purifying a fusion protein include Arg-tag, FLAG-tag, Strep-tag, an epitope capable of being recognized by an antibody, such as c-myc-tag (recognized by an anti-c-myc antibody), SBP-tag, S-tag, calmodulin binding peptide, cellulose binding domain, chitin binding domain, glutathione S-transferase-tag, maltose binding protein, NusA, TrxA, DsbA, Avi-tag, among others, an amino acid sequence such as Ala-His-Gly-His-Arg-Pro (SEQ ID NO:2); Pro-Ile-His-Asp-His-Asp-His-Pro-His-Leu-Val-Ile-His-Ser (SEQ ID NO:3); Gly-Met-Thr-Cys-X-X-Cys (SEQ ID NO:4); β-galactosidase and the like.

It has been previously disclosed that His-tag, used for the isolation or purification of recombinant TF, has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Thus, in one embodiment, the tag is a His-tag. In another embodiment, such His-tag is bound to the C-terminal domain of the TF protein first portion. In still another embodiment, such His-tag is bound to the N-terminal domain of the TF protein first portion.

In another embodiment, the first portion of the fusion protein comprises the mature TF protein, preferably, the mature human TF protein. In still another embodiment, the first portion is the human mature TF.

In another embodiment, the fusion protein has a first portion which comprises the mature human tissue factor protein with at least one of the N-glycosilation sites being non-functional, and a second portion which comprises a His-tag. In a preferred embodiment, the tissue factor is a fusion protein comprising a human TF of SEQ ID NO: 5 which: (a) lacks the signal sequence, (b) has a N124A mutation at the glycosilation site, and (c) has an hexahistidine tag at the C terminus.

Said fusion protein may be obtained by conventional means, e.g., by means of gene expression of the nucleotide sequence encoding for said fusion protein in a suitable yeast cell. The eventual tag can be used, if desired, for the isolation or purification of said fusion protein.

The term "lipidated Tissue Factor (TF)" as used herein refers to any source of TF being this TF totally or partially inserted in lipidic vesicles or cellular membranes. Illustrative, non-limiting examples of "lipidated TF" sources are: 1) lipidated TF containing tissue extract (whose isolation can be carried out from several tissues such as cerebral, placental and lung tissue, tissues from different animals such as sheep, cows, rabbits, dogs, and human beings, among others); 2) purified and (re)-lipidated TF proteinaceous component, i.e. what the lipid component (phospholipids) has been added to after TF purification; and 3) lipidated TF containing cell extract where lipidic fraction comes from the host cell and TF has been recombinantly expressed.

Purified and (re)-lipidated TF can be prepared, by way of an illustrative, non-limiting example, according to the protocol previously described by Mimms L. T. et al., "Phospholipid vesicle formation and transmembrane protein incorporation using octyl glucoside", Biochemistry, 1981, vol. 20(4), p. 833-840. In said protocol, non-lipidated TF is incorporated within phospholipid vesicles using a non-ionic detergent, such as N-octyl-beta-D-galactopyranoside, for example. The phospholipids that can be used in lipidated TF according to the invention may have any origin (animal, plant or synthetic). Virtually any phospholipid can be used in the preparation of the lipidated TF referred herein. Illustrative, non-limiting examples of phospholipids that can be used in the preparation of lipidated TF include phosphatidylcholine, phosphatidylserine, phosphatidylethanolamine, etc. The TF proteinaceous component:phospholipid ratio (molar, weight or volumetric) may vary within a wide range, for example, from about 1:50000 to about 1:3000. In a particular embodiment the lipidated TF is a lipidated human TF and consists of the proteinaceous component of a TF isolated from human tissue, or from recombinant cells, and inserted in a lipid membrane in a suitable TF proteinaceous component:phospholipid ratio. The preferred molar ratios of lipid composition (preferible phosphatydylcholine and phosphatydylserine) to achieve the optimal TF activity is well known in the state of the art.

Concerning the preparation of lipidated TF containing cell extract where lipidic fraction comes from the host cell and TF has been recombinantly expressed, since the TF molecule contains a large hydrophobic domain, it is reasonable to assume that cloning of rTF gene in any of the available eukaryotic expression systems would result in the expression or recombinant TF (rTF) protein associated to host cells lipidic membranes. In this association it would be possible that host membranous components could mimic or provide similar or identical scaffold to the rTF molecules as those found in mammalian cells to witch TF normally associates. Thus, purification of these mermbranous components would result in an available source of lipidated rTF. Due to its chemical composition, these isolated lipidic membranous components would be found predominantly in the form of vesicles of different sizes. In addition to rTF those vesicles would also contain specific proteins or lipids depending on the origin of the host cell. Therefore, the lipidic membranes coming from yeast, bacteria, cells from different origin such as insect, mammal, plant, fish, algae, would contain also proteins and lipids characteristics of such host cells).

TF comprises a hydrophobic region (the transmembrane domain) which is anchored in a lipid membrane, whereas the hydrophilic regions thereof (i.e., the amino-terminus region and the carboxyl-terminus region of said TF protein) face the exoplasmic side of the membrane.

In the present invention, the term "lipid membrane" has to be understood as an organized layer of a few molecules (lipids and proteins) thick forming the boundary of a cell (i.e., the cell or plasma membrane) or the boundaries of intracellular organelles. Typically a membrane is composed of two oriented lipid layers in which proteins can be embedded. A lipid bilayer, which is the basic structure of the membranes of a cell, is usually formed by amphipathic molecules (e.g. phospholipids, fatty acids etc.) in an aqueous environment, each molecule being oriented with the hydrophilic group on the outside of the layer and the hydrophobic group to the interior of the layer. Preferably, the TF is anchored in a lipid microvesicle.

In the present invention, "lipid microvesicle" has to be understood as a small and closed compartment, which is substantially composed by lipids mono or bilayers. The size of the TF-bearing microvesicle of the invention can vary within a relatively broad range, usually, said size is equal to or lower than 10 µm, typically equal to or lower than 0.5 µm. In a particular embodiment, the size of the TF-bearing yeast derived microvesicles of the invention range from 10 to 0.01 µm. The microvesicles are formed by lipid membranes, or fragments thereof, from eukaryotic cells. In one embodiment, the microvesicle can be enriched in negatively-charged phospholipids, preferably, phosphatidilserine. Methods for enriching such microvesicles in phosphatidilserine are disclosed, for instance, in WO2011/131658. Briefly, said TF-bearing microvesicles enriched in phospholipids can be prepared by a process comprising: a) subjecting a culture of recombinant eukaryotic cells expressing TF protein to fermentation under conditions which allow the expression of said TF protein, or fragment thereof having pro-coagulant activity; b) pelleting the cultured cells resulting from the fermentation of step a), to render a fermentation product; c) subjecting said fermentation product from step b) to homogenization, to render a fermentation homogenate; and d) subjecting said fermentation homogenate from step c) to separation, to render a pellet and a clarified yeast extract (CYE) containing said TF-bearing derived microvesicles having pro-coagulant activity; e) collecting said clarified yeast extract (CYE) containing said TF-bearing yeast derived microvesicles having pro-coagulant activity; and, optionally, f) if desired, isolating or purifying said TF-bearing yeast derived microvesicles having pro-coagulant activity by size partitioning procedures, and g) enrichment of the obtained microvesicles in negatively charge phospholipid (i.e. phosphatydilserine) by the incubation of both components phosphatidylserine and lipid microvesicles containing TF As a step previous to the mentioned incubation, phosphatydilserine could be prepared as multilamelar vesicles by resuspension of lipids in a buffered solution followed by sonication.

The method of production of TF depends on the eukaryotic cell used. Generally, the eukaryotic cell is transformed with a expression vector comprising the nucleotide sequence coding for TF protein operatively linked to a functional promoter in a cell which can be from: fungi, yeast, plant or animal (such as fish, reptilian, mammalian, insect, etc). The cDNA coding for TF protein can be amplified by the polymerase chain reaction (PCR) using a cDNA library as template and the appropriate primers. Example 1 discloses the amplification of the cDNA coding for the mature hTF protein with 18 extra nucleotides (coding for six histidines) at the 3' end.

In another embodiment, the TF protein could be purified from steps a) to d) above, following well established chromatography purification procedures. Once purified, the TF protein could be lipidated in vitro with optimal phospholipid concentrations.

In an additional embodiment, lipidated TF could be obtained from animal tissue extracts, such as cerebral, placental and lung tissue,and tissues from different animals such as sheep, cows, rabbits, dogs, and human beings, among others. Preferably, the lipidated TF is from human being. More preferably, the lipidated TF is human recombinant.

In the present invention, the term "thrombin" (FIIa) has to be understood as the key serine protease that plays a pivotal role in hemostasis. FIIa activates many of the coagulation factors involved in the coagulation cascade including Fibrinogen (FI), Factor V (FV), Factor VIII (FVIII), Factor XI (FXI), and Factor XIII (FXIII). FIIa also activates platelets and vascular endothelial cells. The thrombin precursor is the zymogen Prothrombin (FII), which is secreted into blood from the liver. During the coagulation cascade, FII is converted into FIIa at different rates first by activated FX (FXa), and at the latest steps of the cascade by the prothrombiase complex (FXa:FVa).Commercial FIIa is produced after isolation of FII from plasma of human or bovine origin. After its isolation, FII is transformed in vitro into FIIa. All current marketed FSs contain FIIa from human or bovine origin. Additionally, highly purified recombinant human FIIa is also available. This recombinant human FIIa is obtained after purification of human FII from recombinant Chinese Hamster Ovary (CHO) cells, followed by its processing to FIIa by in vitro procedures.

Exemplary FII proteins that can be used in the present invention include human FII (UniProtKB/Swiss-Prot. Version 182, November 2012, accession number P00734.), bovine FII (UniProtKB/Swiss-Prot.Version 153, November 2012, accession number P00735), mouse FII (UniProtKB/Swiss-Prot. Version 141, November 2012, accession number P19221), pig FII (UniProtKB/SwissProt. Version 37, November 2012. accession number B3STX9), and FII proteins of different animals. Preferably, the thrombin is from human or bovine origins, although more preferably is human thrombin and even more preferably recombinant human thrombin.

It is well-established in the state of the art that the concentration of thrombin is provided in units per mL. The units for expressing the amount of thrombin can be "NIH" units or international units. It is also well-established in the state of the art how the conversion of the units expressed in one system has to be performed into the other:
1 IOWA unit = 0.83 NIH unit
1 IU = 1 NIH unit
1 NIH unit = 0.324 ± 0.073 mg
1 NIH unit = 1 USP unit

In one embodiment, any of the compositions of the present invention is in liquid form. In this embodiment, the composition can be obtained preparing separate solutions of thrombin and lipidated TF solution in such concentrations that, when both solutions are mixed, the resulting composition has thrombin and TF in the specific concentrations mentioned above. Alternatively, the composition can be obtained preparing a solution of thrombin or lipidated TF, and the other component is added in powder form, provided that the final concentration of each of the components, in the resulting liquid composition, is the one mentioned above. Any physiologically acceptable solvent can be used. Illustrative non-limitative examples of solvents include water, saline buffer, phosphate buffer, or a bodily fluid, such as blood or synovial fluid.

In another embodiment, any of the compositions of the invention is in powder form. In this embodiment, the composition can be obtained starting from a liquid composition (which can be obtained as disclosed above) which already has thrombin and lipidated TF in the specific concentrations and then, it can be subjected to well-known powder techniques, provided that such techniques do not adversely affect to the stability of thrombin or TF. Illustrative non-limitative examples of powder techniques are based on desiccation techniques, like i.e. lyophilization, or spray drying.

Alternatively, the powder composition can be obtained starting from thrombin and lipidated TF in powder form. In this embodiment, the skilled person can easily determine the amount of each one of the components needed to obtain a composition with each of the components at the specified concentrations.

In another embodiment, any of the compositions of the invention is in the form of a solid foam. These solid foams can be obtained from a liquid composition (which can be obtained as disclosed above) by spray drying techniques.

In another embodiment, any of the compositions of the invention is in semi-solid form. In order to obtain such form, appropriate excipients and carriers are needed. Below are provided excipients and carriers useful in obtaining such semi-solid compositions.

In one embodiment of the first aspect of the invention, the composition further comprises a cross-linking agent.

In another embodiment of the first aspect of the invention, the composition further comprises a fibrinolysis inhibitor.

In still another embodiment of the first aspect of the invention, the composition further comprises CaCl₂.

In a further embodiment of the first aspect of the invention, the composition further comprises a cross-linking agent, and a fibrinolysis inhibitor.

In a further embodiment of the first aspect of the invention, the composition further comprises a cross-linking agent, and CaCl₂.

In a further embodiment of the first aspect of the invention, the composition further comprises a fibrinolysis inhibitor, and CaCl₂.

In a further embodiment of the first aspect of the invention, the composition further comprises a cross-linking agent, fibrinolysis inhibitor, and CaCl₂.

Preferably, said cross-linking agent is FXIII or activated FXllla.

Preferably, said fibrinolysis inhibitor is selected from aprotinin, tranexamic acid, and aminocaproic acid.

As mentioned above, in a further aspect the present invention provides a pharmaceutical or veterinary composition comprising a therapeutically effective amount of the composition of the first aspect of the invention, together with appropriate pharmaceutically or veterinary acceptable excipients and/or carriers.

The expression "therapeutically effective amount" as used herein, refers to the amount of the composition that, when administered, is sufficient to prevent development of, or alleviate to some extent, one or more of the symptoms of the disease which is addressed. The particular dose of the composition administered according to this invention will of course be determined by the particular circumstances surrounding the case, including the compound administered, the route of administration, the particular condition being treated, and the similar considerations.

Processes for the preparation of such pharmaceutical or veterinary compositions are well-known in the state of the art.

The pharmaceutical or veterinary composition of the invention should be applied topically to the bleeding site, either directly or in a carrier material, such as a gauze. Examples of topical application includes, but are not limited to, external, cutaneous, intranasal, intravesicular, intravaginal, buccal, rectal, ophthalmic, endoscopic topical application, and instillation.

The pharmaceutical or veterinary composition of the second aspect of the invention can take the form of soft or hard gel, liquid, lotion, cream, ointments, pastes, roll-on formulations, sprays, aerosols, foams, pad-applied formulations and film-forming formulations.

For the topical administration, appropriate pharmaceutical excipients or carriers include, but do not limit to, hydrating agents, emollients, emulsifiers, humectants, pH-regulating agents, antioxidants, preservative agents, vehicles, or mixtures thereof. The excipients or carriers used have affinity for the skin, are well tolerated, stable, and are used in an amount adequate to provide the desired consistency, and ease application.

When the pharmaceutical or veterinary composition of the present invention is topical, it can be formulated in several forms that include, but are not limited to, solutions, lotions, gels, ointments, pastes, creams, of soft or hard gel, liquid, foams, roll-on formulations, sprays, aerosols, pad-applied formulations and film-forming formulations. These topical pharmaceutical compositions can be prepared according to methods well known in the state of the art. The appropriate pharmaceutical excipients and/or carriers, and their amounts, can readily be determined by those skilled in the art according to the type of formulation being prepared.

In one embodiment of the pharmaceutical or veterinary composition of the second aspect of the invention, the composition comprises a cross-linking agent.

In another embodiment of the pharmaceutical or veterinary composition of the second aspect of the invention, the composition further comprises a fibrinolysis inhibitor.

In still another embodiment of the pharmaceutical or veterinary composition of the second aspect of the invention, the composition further comprises CaCl₂.

In a further embodiment of the pharmaceutical or veterinary composition of the second aspect of the invention, the composition further comprises a cross-linking agent and a fibrinolysis inhibitor.

In a further embodiment of the pharmaceutical or veterinary composition of the second aspect of the invention, the hemostatic composition further comprises a cross-linking agent and CaCl₂.

In a further embodiment of the pharmaceutical or veterinary composition of the second aspect of the invention, the composition further comprises a fibrinolysis inhibitor and CaCl₂.

In a further embodiment of the pharmaceutical or veterinary composition of the second aspect of the invention, the composition further comprises a cross-linking agent, fibrinolysis inhibitor and CaCl₂.

Preferably, said cross-linking agent is FXIII.

Preferably, said fibrinolysis inhibitor is selected from aprotinin, tranexamic acid, and aminocaproic acid.

The compositions of the invention, can be used as an adjunct to hemostasis in patients undergoing surgery when control of bleeding by conventional surgical techniques (such as suture, ligature, and cautery), as well as adjunct in standard surgical techniques (such as suture and ligature) to prevent leakage.

In a further aspect, the present invention provides hemostatic compositions comprising the composition of the first aspect of the invention or the pharmaceutical or veterinary composition of the second aspect of the invention, and a carrier material.

In the present invention, the term "hemostatic" referred to compositions, effect and use, has to be understood as a substance that promotes hemostasis, that is, that stops bleeding. It can also be referred as "antihemorrhagic" and "procoagulant".

The term "carrier material" has to be understood as a substrate of suitable material allowing depositing the composition of the invention thereon, its transport and its release at the desired site, for example, in the site where the compositions of the invention has to exert its therapeutic effect. Said carrier material can be a solid support. Illustrative, non-limiting examples of solid supports include dressings, band-aids, compresses, plasters, etc. Illustrative, non-limiting examples of liquid supports include gels, sprays, mouthwashes, etc. The interaction between the components of the composition and the solid material can be a physical or chemical interaction.

In one embodiment, the carrier material is a solid support made of collagen, gelatin or oxidized regenerated cellulose.

In another embodiment, the carrier material is a device for the dispensing of a powder.

In one embodiment, the composition of the first aspect is applied on the carrier material in liquid form, i.e., the support is impregnated with the composition of the present invention.

In another embodiment of the hemostatic composition of the third aspect of the invention, the hemostatic composition comprises a cross-linking agent.

In another embodiment of the hemostatic composition of the third aspect of the invention, the hemostatic composition further comprises a fibrinolysis inhibitor.

In still another embodiment of the hemostatic composition of the third aspect of the invention, the hemostatic composition further comprises CaCl₂.

In a further embodiment of the hemostatic composition of the third aspect of the invention, the hemostatic composition further comprises a cross-linking agent and a fibrinolysis inhibitor.

In a further embodiment of the hemostatic composition of the third aspect of the invention, the hemostatic composition further comprises a cross-linking agent and CaCl₂.

In a further embodiment of the hemostatic composition of the third aspect of the invention, the hemostatic composition further comprises a fibrinolysis inhibitor and CaCl₂.

In a further embodiment of the hemostatic composition of the third aspect of the invention, the hemostatic composition further comprises a cross-linking agent, fibrinolysis inhibitor and CaCl₂.

Preferably, said cross-linking agent is FXIII.

Preferably, said fibrinolysis inhibitor is selected from aprotinin, tranexamic acid, and aminocaproic acid.

Due to the properties pointed out in detail above, the compositions of the invention can be used as a wound healing agent.

The expression "wound healing" relates to an intricate process in which the skin (or some other organ) repairs itself after injury wound healing of any kind and at any site. It can be normal and impaired wound healing. The latter is found in particular in the case of diseases, such as diabetes mellitus, vasculitis, arterial occlusive disease, chronic venous and/or infected ulcer as well as poorly healing gastric ulcer. Impaired wound healing is also found in the case of innervations impairment such as paraplegia, leprosy, neuropathy, etc., and decubital gangrene of persons in need of care. Impaired wound healing will also be given if weak sutures and impaired healing occur after operations, particularly of the intestines and transplantations of skin and other organs, respectively.

Impaired wound healing is also found in the case of bone fractures, burns, and treatments using steroids.

As used herein, the term "wound" includes an injury to any tissue, including for example, delayed or difficult to heal wounds, and chronic wounds. Examples of wounds may include both open and closed wounds. The term "wound" may also include for example, injuries to the skin and subcutaneous tissue initiated in different ways (e.g., pressure sores from extended bed rest and wounds induced by trauma) and with varying characteristics. Wounds may be classified into one of four grades depending on the depth of the wound: i) Grade I wounds limited to the epithelium; ii) Grade II wounds extending into the dermis; iii) Grade III wounds extending into the subcutaneous tissue; and iv) Grade IV (or full-thickness wounds) wounds wherein bones are exposed (e.g., a bony pressure point such as the greater trochanter or the sacrum).

The term "chronic wound" generally refers to a wound that has not healed. Chronic wounds include venous ulcers, venous stasis ulcers, arterial ulcers, pressure ulcers, diabetic ulcers, diabetic foot ulcers, vasculitic ulcers, decubitus ulcers, burn ulcers, trauma-induced ulcers, infectious ulcers, mixed ulcers, and pyoderma gangrenosum.

The compositions of the invention can also be used in the treatment of hemorrhages. Preferably, the compositions of the present invention are applied topically. Such hemorrhages can be due to coagulopathies, injuries, wound, or platelet disorders, among others.

As it is shown below, the compositions of the invention act as antihemorrhagic agent, and, consequently, can be used to treat or correct hemorrhagic disorders, particularly those hemorrhagic disorders associated with hemorrhagic diathesis.

The term "hemorrhagic diathesis" refers to the process causing a hemostasic disorder and which, as a result, gives rise to the occurrence of a hemorrhagic syndrome which may occasionally occur with extended and excessive bleeding.

The term "coagulopathy" refers to a coagulation factor disorder. This disorder may be due to a specific coagulation factor deficiency or deficit, the consequence of which will be the occurrence of a hemorrhagic syndrome, or due to a coagulation factor disorder. The coagulopathy may generally be a congenital coagulopathy or an acquired coagulopathy. As illustrative, non-limiting examples of congenital coagulopathies, deficiencies of coagulation factors selected from coagulation Factor V (FV), coagulation Factor VII (FVII), coagulation Factor VIII (FVIII), the deficit or deficiency of which causes hemophilia A, coagulation Factor IX (FIX) the deficit or deficiency of which causes hemophilia B, coagulation Factor X (FX), coagulation Factor XI (FXI) the deficit or deficiency of which causes hemophilia C, coagulation Factor XII (FXII), coagulation Factor XIII (FXIII) and their combinations, can be mentioned. Acquired coagulopathies may have different origins. Illustrative examples include coagulation factor synthesis deficiencies in severe hepatic failure, anticoagulant therapy (such as heparin, low molecular weight heparins, warfarin, coumarin derivatives, dicoumarins, etc.). An alternative mechanism is based on an exaggerated consumption of coagulation factors such that they are not available to form the clot in a bleeding lesion. This mechanism occurs in the disseminated intravascular coagulation syndrome or coagulopathy due to consumption occurring in multiple illnesses such as in severe sepsis damaging the microcirculation endothelium activating platelets and coagulation factors with the formation of multiple microthrombi; in blood invasion by TF such as placental release; in the retention of a dead fetus; in multiple traumas with the crushing of tissues; in poisonous snake bites, etc. In vasculitis, parietal and endothelial damage releases coagulation activators. The consumption of coagulation factors is worsened by lysis of the fibrin of numerous microthrombi due to the action of plasmin, which are antiplatelets and anticoagulants.

The term "platelet disorder" refers to a disorder both in the number and in functional ability of platelets, the result of which is the occurrence of a hemorrhagic syndrome. Said platelet disorder may be congenital or acquired. In a particular embodiment, said platelet disorder is a congenital platelet disorder. Illustrative, non-limiting examples of congenital platelet disorders include Glanzmann's disease, Bernard Soulier disease, Bolin-Jamieson syndrome, Wiskott-Aldrich syndrome, Paris-Trousseau-Jacobsen syndrome, X chromosome thrombocytopenia, Gray platelet syndrome, Sebastian syndrome and Fanconi anemia. In another particular embodiment, said platelet disorder is an acquired platelet disorder. Illustrative, non-limiting examples of acquired platelet disorders incluye myeloproliferative disorders, such as thrombocythemia, polycythemia, chronic myelocytic leukemia, etc.; there are functional platelet disorders in myeloid metaplasia with increased bleeding time, glass bead retention defects, platelet aggregation defect, abnormal release, and platelet factor III defect. Functional platelet defects have been found in dysproteinemias in scurvy and in congenital heart disease and cirrhosis.

In one embodiment of the seventh aspect of the invention, it is provided the compositions as defined above for use in the topical treatment of hemorrhages by its application to the injury site.

The term "subject" as used herein includes any member of an animal species, including the human species; by way of an illustrative, non-limiting example, said subject can be a mammal, such as a primate, a domestic animal, a rodent, etc. Said subject is preferably a man or woman of any age and race. In a particular embodiment, said subject is a human being with no history of hemostasis disorders, such as an individual having no coagulopathies or platelet disorders. In another particular embodiment, said subject is a human being having a history of hemostasis disorders, such as an individual having hemorrhagic diathesis, for example, a coagulopathy, such as a congenital or acquired coagulopathy, or a platelet disorder, such as a congenital or acquired platelet disorder.

Since TF is a well known pro-angiogenic molecule, the compositions of the invention can also be used in the treatment of a disease associated to a deficient angiogenesis.

The term "disease associated to deficient angiogenesis", as used herein, relates to diseases which can be cured by activating vessel formation. The expression "vessel formation" relates to a vessel formation of any kind and at any site. The promotion of vessel formation may be useful in a number of clinical conditions. For example, the composition of the invention may be used to promote angiogenesis of collateral vasculature in myocardial tissue during or following ischaemic disease, myocardial infarction or following coronary bypass surgery. Other diseases or conditions which may be treated by the provision of the compositions of the invention include vascular disease and/or ischaemic disease causing pathology of the peripheral or central nervous system. Such conditions/diseases may include cerebrovascular accidents, e.g. caused by clot occlusions or by rupture of aneurysms, or general/localised ischemia causing neuronal death or peripheral functional impairment such as in motor or sensory functions or speech impairment, ischemic cardiomyopathy, or peripheral arterial disease, such as chronic limb ischemia claudication (skeletal muscle), rest pain/ischemic ulceration/gangrene. Moreover, the promotion of vessel formation is adequate for replacing impaired, e g., old, blood vessels. They can be present, e.g., in the brain or heart, so that an apoplexy or infarction can be prevented or treated. Precautions can also be taken against presbyphrenia. In addition, it relates to a vessel formation for treating arteriosclerosis, Crohn's disease and ulcerative colitis, diabetic retinopathy and deep venous thrombosis of the legs/ulcus cruris as well as the prevention of relapses.

Finally, in a further aspect the present invention provides kits comprising the compositions of the first or second aspect of the invention together with an applicator.

The kit can take the form of (a) two-parts, in such a way that each part contains each of the components forming the combination, that is one part of the kit contains the thrombin, and the other one contains the lipidated tissue factor; or, alternatively, (b) as one-part, in such a way that both, thrombin and lipidated TF shares a single part.

Thus, in one embodiment, the kit comprises: a) a first part comprising thrombin; b) a second part comprising the lipidated tissue factor; and c) an applicator to apply simultaneously all the components included in the kit.

In another embodiment, the kit comprises: a) a first part comprising thrombin and lipidated tissue factor; and b) an applicator an applicator to apply simultaneously all the components included in the kit.

The thrombin, and lipidated tissue factor contained in the kit can be in any suitable form, such as in solution, suspension, or powder, provided that they are in the concentrations of 1-30 IU/g composition and 5-150 ng/g composition, respectively, together with the appropriate pharmaceutically or veterinary acceptable excipients and/or carriers.

In addition, the kit can include instructions for its use in any of the applications mentioned above.

In one embodiment, the kit further comprises a cross-linking agent.

In another embodiment, the kit further comprises a fibrinolysis inhibitor.

In still another embodiment, the kit further comprises CaCl₂.

In a further embodiment, the kit further comprises a cross-linking agent and a fibrinolysis inhibitor.

In a further embodiment, the kit comprises a cross-linking agent, and CaCl₂.

In a further embodiment, the kit comprises a fibrinolysis inhibitor, and CaCl₂. In a further embodiment, the kit comprises a cross-linking agent, fibrinolysis inhibitor, and CaCl₂.

Preferably, said cross-linking agent is FXIII.

Preferably, said fibrinolysis inhibitor is selected from aprotinin, tranexamic acid, and aminocaproic acid.

The cross-linking agent, the fibrinolysis inhibitor, and/or CaCl₂, can be included in any one of the parts identified above for thrombin, and lipidated tissue factor, or alternatively, each one can be in further separate parts or can be sharing the same part of the kit.

Any further component can be included in the kit provided that it does not negatively effect to the hemostatic profile of the composition included therein. Such extra-components can be included in any one of the parts identified above for thrombin and lipidated tissue factor. Alternatively, each one of the "extra-components" of the kit can be in further separate parts.

Illustrative non-limiting examples of applicators that allow applying simultaneously all the components included in the kit are barreled syringes, spray applicators, a pad, or a device for dispensing the components when they are in powder form, among others.

An example of dispensing device of powder is the one disclosed in WO2010/07033.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1: Production of the recombinant tissue factor based on the expression of the full-length TF his-tag modified protein in east hereinafter also referred as "TT-173")

The yeast episomal vector pTT-1 0301 described in Example 1 of WO2008080989 which comprises the URA3 gene, the ampicillin resistance gene, the yeast 2 µ origin of replication, the glyceraldehyde-3-phosphate dehydrogenase (GPD) promoter and the yeast transcription termination signal of the phosphoglycerate kinase, was used to clone, under the control of the GPD promoter, a cDNA of SEQ ID NO: 6 coding for the mature hTF protein (aa 33-295 of SEQ ID NO:1) with the His-tag at the 3' end and an Asn124Ala mutation which inactivates one of the potential N-glycosylation sites in the native hTF sequence (SEQ ID NO: 5).

SEQ ID NO: 6 was obtained as follows. The human cDNA sequence of TF (Genbank accession number BC011029, SEQ ID NO: 11) was amplified using the primers of sequence SEQ ID NO: 7 and SEQ ID NO: 8. The resulting amplified DNA sequence was further modified by site-directed mutagenesis, using primers of sequence SEQ ID NO: 9 and SEQ ID NO: 10. For this, the Quick Change site-directed mutagenesis kit (Stratagene) was used, following the method described in Stratagene manuals. With such latter mutagenesis, the TF sequence containing the Asn to Ala point mutation at amino acid residue 124 of the TF protein sequence was generated.

After transformation, yeast strains capable of growing in uracil-free media were collected and tested for their ability to express hTF by Western-blot analysis of the yeast extracts essentially as described in WO2008080989. Briefly, yeasts, containing the episomal expression plasmid with a modified version of the TF coding gene (SEQ ID NO: 6), are grown in a fermentor, and the resulting cells collected by centrifugation. The collected cells are resuspended in an appropriate lysis buffer (20 mM phosphate, 50 mM NaCl, pH 7.4), and subjected to high pressure lysis by passing three times through a homogenizer at 1000 bars of pressure to obtain the fermentation lysate (fermentation homogenate). The cell suspension is maintained cold by sumerging the container in an ice bath. After that, the heaviest material is eliminated from the fermentation lysate by means of centrifugation, thus obtaining a clarified yeast extract (CYE) supernatant. The CYE obtained from the homogenization step is diluted with lysis buffer until total protein content reach a concentration between 5-6 mg/mL, and filtered through consecutive filters of 0.45 µm, 0.2 µm, and 0.1 µm respectively to reduce the amount of non-membrane host cell derived proteins (HCP) and to obtain a more homogeneous material in rTF-vesicles.

The vesicles in the microfiltrate retentate of 0.1 µm have diameters below 0.2 µm. To eliminate traces of DNA, the microfiltrate retentate of 0.1 µm is then treated with 10 U/mL of the endonuclease Benzonase (Merck) and magnesium chloride to a final concentration of 1 mM, incubated for 12 h at 4°C in a glass container rocking at approximately 20 rpm, and used for further enrichment steps.

To further fractionate different vesicle subpopulations according to their size, and to remove unwanted residual cell material, the filter-clarified yeast extract is applied to a Sephacryl size-exclusion chromatography column. After separation, fractions 2 to 22, corresponding to vesicles enriched in rTF, are pooled and filter-sterilized. After that, phosphatydylserine (PS) at a final concentration of 0.1 mg/mL is added to the sterilized product and the mixture is maintained at room temperature for 2 h to allow the incorporation of PS into the vesicles. As a previous step, PS could be prepared as multilamelar vesicles by resuspension of lipids in a buffered solution followed by sonication, following the Morrissey Lab Protocol for Preparing Phospholipid Vesicles (SUV) by sonication. Briefly: (1) Dispense 2.6 µmole total phospholipids (PL) in a glass test tube (a 13 x 100 mm tube is a convenient size); (2) In the fume hood, dry the PL mixture under a gentle stream of nitrogen or argon. When dry, speed-vac for an additional 60 minutes under high vacuum. (This is to remove any residual chloroform.); (3) To the dried-down PL, add 2.6 ml room temperature HBS solution and cover the end of the tube with parafilm. Let sit 1 hr at room temperature; (4) Vortex tube vigorously to completely resuspend the PL. The result should be a milky, uniform suspension; (5) Fill the bath sonicator with room temperature water. Using a ring stand and test tube clamp, suspend the tube containing the PL suspension in the bath. The liquid level inside the tube should equal that outside the tube. Sonicate until the suspension changes from milky to nearly clear (i.e., only very slightly hazy) in appearance. Check every 10 min; it will usually take between 10 and 30 min total sonication time. (Be sure not to let the bath overheat, and do not drain the bath until it has completely cooled.); and (6) Store the final product at 4 degrees C. The result is a suspension of small unilamellar vesicles (SUV) containing a total of 1 mM phospholipid in HBS.

After 2 h of incubation, the sterile product, containing PS enriched vesicles, is aliquoted and lyophilized. This lyophilized material corresponds to the rTF used in the tests below and consists of a protein of SEQ ID NO:5 anchored to a lipidic vesicle highly enriched in PS.

### Example 2: Effect of the addition of TF over thrombin in whole blood

For these experiments sodium citrate treated blood samples from three healthy individuals were used. Prior to start the experiments, thrombin and lipidated TF solutions were prepared. For this, one vial containing 1000 NIH units of lyophilized bovine thrombin (Ref. T4648 SIGMA) was resuspended in 1 mL of Phosphate buffer (20mM Phosphate, 50mM NaCl, pH 7.4). In parallel, lyophilized TT-173 containing 1 µg of lipidated TF was also reconstituted in 1 mL of Phosphate buffer. From these two stock solutions, working solutions containing:
i) individual test items: thrombin (1000, 300, 30, 3, and 1 IU/mL and) or lipidated TF (150, 15, and 5 ng/mL), and
ii) a combination of thrombin (IU)+ lipidated TF(ng) per milliliter, at the following ratios: 1 +5, 1+15, 1 +150, 3+5, 3+15, 3+150, 30+5, 30+15 and 30+150.
were prepared.

It was found, for each one of the solutions prepared, that either the individual test items, or the combinations, that one mL weighted 1 gram. Therefore, the concentrations stated above for: thrombin, lipidated TF or the different thrombin+lipidated TF ratios, could be expressed also per gram of solution (in case of thrombin or lipidated TF alone) or per gram of composition (in case of the combinations thrombin+lipidated TF).

Once prepared, 20 µL of the above solutions were placed independently in the specimen cup of a thromboelastograph (TEG), followed by the addition of 20 µL of calcium chloride. Then, 300µL of blood were transferred into the TEG cup and the coagulation time parameter (hereinafter also referred as "CT parameter") was monitored by TEG. Finally, the CT time for each test item (thrombin, lipidated TF or the combination) concentration was recorded. The results are summarized in Tables 1-2:

**Table 1: CT when thrombin alone is used**

| Amount of thrombin | CT in seconds |
|---|---|
| 1 IU/gram Thrombin | 735±21.2 |
| 3 IU/gram Thrombin | 506±38.2 |
| 30 IU/gram Thrombin | 127.5±40.3 |
| 300 IU/gram Thrombin | 84.7±20.1 |

**Table 2: CT when thrombin+lipidated TF compositions are used**

| Combinations Thrombin+Lipidated TF (ratios expressed per gram of composition) | | CT in seconds |
|---|---|---|
| Thrombin (IU) | Lipidated TF (ng) | |
| 0 | 0 | 955.5±119.5 |
| 1 | 5 | 157.5±0.7 |
| 1 | 15 | 136±11.3 |
| 1 | 150 | 89±5.6 |
| 3 | 5 | 160±1.4 |
| 3 | 15 | 125±4.2 |
| 3 | 150 | 85±9.8 |
| 30 | 5 | 105.5±3.5 |
| 30 | 15 | 107.5±14.8 |
| 30 | 150 | 87±21.2 |

As expected, the addition of exogenous thrombin to blood at concentrations of 1, 3, 30 IU units /g of combination significantly reduces the CT value in a dose dependent manner (see Table 1).

The next step was to determine which effect exerted the combination of lipidated TF to the tested concentrations of FIIa (1, 3 and 30 U/mL). Surprisingly, the addition of minimal amounts of lipidated TF (5 ng/mL) to FIIa induced a remarkable reduction in the CT time. As shown in Table 2, a combination of 5 ng of lipidated TF with 1, 3, or 30 IU of thrombin induces a

CT time similar to the one obtained with 300 U/mL of thrombin alone.

From these results, it can be concluded that a composition comprising 1-30 IU of thrombin and 5-150 ng of lipidated TF, per gram of composition, results in a synergistic clotting effect.

### REFERENCES CITED IN THE APPLICATION

WO2008/080989;
WO2010/07033;
WO2011/131658; and
Mimms LT, Zampighi G, Nozaki Y, Tanford C, Reynolds JA. Phospholipid vesicle formation and transmembrane protein incorporation using octyl glucoside.Biochemistry. 1981 Feb 17;20(4):833-40

## Claims

1. A composition comprising a combination of lipidated tissue factor and thrombin, wherein the amount of thrombin is comprised from 1 to 30 IU per gram of composition, and the amount of lipidated tissue factor is comprised from 5 to 150 ng per gram of composition.

2. The composition according to claim 1, wherein the lipidated tissue factor is comprised from 5 to 15 ng per gram of composition.

3. The composition according to any one of the preceding claims, wherein at least one of the N-glycosilation sites of the tissue factor is non-functional.

4. The composition according to any one of the claims 1-3, wherein said tissue factor is a fusion protein comprising a first portion which comprises the tissue factor protein, and a second portion which comprises another peptide or protein.

5. The composition according to claim 4, wherein the second portion is a tag.

6. The composition according to any one of the claims 4-5, wherein the fusion protein has a first portion which comprises the mature human tissue factor protein with at least one of the N-glycosilation sites being non-functional, and a second portion which comprises a His-tag.

7. The composition according to claim 6, wherein the fusion protein corresponds to SEQ ID NO: 5.

8. The composition according to claim any one of the claims 1-7, wherein the TF protein is anchored in a lipid microvesicle.

9. A pharmaceutical or veterinary composition comprising a therapeutically effective amount of the composition according to any one of the claims 1-8, together with other appropriate pharmaceutically or veterinary acceptable excipients and/or carriers.

10. A hemostatic composition comprising the composition according to any one of the claims 1-8 or the pharmaceutical or veterinary composition according to claim 9, and a carrier material.

11. Use of the composition according to any one of the claims 1-8 or of the pharmaceutical or veterinary composition according to claim 9, as a hemostatic agent.

12. A composition according to any one of the claims 1-8 or a pharmaceutical or veterinary composition according to claim 9, or a hemostatic composition according to claim 10, for use as a medicine.

13. A composition according to any one of the claims 1-8 or a pharmaceutical or veterinary composition according to claim 9, or a hemostatic composition according to claim 10, for use in the treatment of haemorrhages.

14. A kit comprising the composition according to any one of the claims 1-8 or the pharmaceutical or veterinary composition according to claim 9 or the hemostatic composition of claim 10, and an applicator that allows the simultaneous application of all the components forming the combination.


SEQUENCE LISTING

<110>  Thrombotargets Europe SL

<120>  Hemostatic Compositions

<130>  P2553EP00

<160>  11

<170>  PatentIn version 3.5

<210>  1
<211>  263
<212>  PRT
<213>  Homo sapiens

<400>  1

Ser Gly Thr Thr Asn Thr Val Ala Ala Tyr Asn Leu Thr Trp Lys Ser
1               5                   10                  15

Thr Asn Phe Lys Thr Ile Leu Glu Trp Glu Pro Lys Pro Val Asn Gln
            20                  25                  30

Val Tyr Thr Val Gln Ile Ser Thr Lys Ser Gly Asp Trp Lys Ser Lys
        35                  40                  45

Cys Phe Tyr Thr Thr Asp Thr Glu Cys Asp Leu Thr Asp Glu Ile Val
    50                  55                  60

Lys Asp Val Lys Gln Thr Tyr Leu Ala Arg Val Phe Ser Tyr Pro Ala
65                  70                  75                  80

Gly Asn Val Glu Ser Thr Gly Ser Ala Gly Glu Pro Leu Tyr Glu Asn
                85                  90                  95

Ser Pro Glu Phe Thr Pro Tyr Leu Glu Thr Asn Leu Gly Gln Pro Thr
            100                 105                 110

Ile Gln Ser Phe Glu Gln Val Gly Thr Lys Val Asn Val Thr Val Glu
        115                 120                 125

Asp Glu Arg Thr Leu Val Arg Arg Asn Asn Thr Phe Leu Ser Leu Arg
    130                 135                 140

Asp Val Phe Gly Lys Asp Leu Ile Tyr Thr Leu Tyr Tyr Trp Lys Ser
145                 150                 155                 160

Ser Ser Ser Gly Lys Lys Thr Ala Lys Thr Asn Thr Asn Glu Phe Leu
                165                 170                 175

Ile Asp Val Asp Lys Gly Glu Asn Tyr Cys Phe Ser Val Gln Ala Val
            180                 185                 190

Ile Pro Ser Arg Thr Val Asn Arg Lys Ser Thr Asp Ser Pro Val Glu
        195                 200                 205

Cys Met Gly Gln Glu Lys Gly Glu Phe Arg Glu Ile Phe Tyr Ile Ile
    210                 215                 220

Gly Ala Val Val Phe Val Val Ile Ile Leu Val Ile Ile Leu Ala Ile
225                 230                 235                 240

Ser Leu His Lys Cys Arg Lys Ala Gly Val Gly Gln Ser Trp Lys Glu
                245                 250                 255

Asn Ser Pro Leu Asn Val Ser
            260

<210>  2
<211>  6
<212>  PRT
<213>  artificial sequence

<220>
<223>  epitope AHGHRP

<400>  2

Ala His Gly His Arg Pro
1               5

<210>  3
<211>  14
<212>  PRT
<213>  artificial sequence

<220>
<223>  epitope

<400>  3

Pro Ile His Asp His Asp His Pro His Leu Val Ile His Ser
1               5                   10

<210>  4
<211>  7
<212>  PRT
<213>  artificial sequence

<220>
<223>  epitope wherein Xaa can be any naturally occurring amino acid

<220>
<221>  misc_feature
<222>  (5)..(6)
<223>  Xaa can be any naturally occurring amino acid

<400>  4

Gly Met Thr Cys Xaa Xaa Cys
1               5

<210>  5
<211>  269
<212>  PRT
<213>  artificial sequence

<220>
<223>  Human mature TF with N124A mutation and C-terminal hexahistidine

<400>  5

Ser Gly Thr Thr Asn Thr Val Ala Ala Tyr Asn Leu Thr Trp Lys Ser
1               5                   10                  15

Thr Asn Phe Lys Thr Ile Leu Glu Trp Glu Pro Lys Pro Val Asn Gln
            20                  25                  30

Val Tyr Thr Val Gln Ile Ser Thr Lys Ser Gly Asp Trp Lys Ser Lys
        35                  40                  45

Cys Phe Tyr Thr Thr Asp Thr Glu Cys Asp Leu Thr Asp Glu Ile Val
    50                  55                  60

Lys Asp Val Lys Gln Thr Tyr Leu Ala Arg Val Phe Ser Tyr Pro Ala
65                  70                  75                  80

Gly Asn Val Glu Ser Thr Gly Ser Ala Gly Glu Pro Leu Tyr Glu Asn
                85                  90                  95

Ser Pro Glu Phe Thr Pro Tyr Leu Glu Thr Asn Leu Gly Gln Pro Thr
            100                 105                 110

Ile Gln Ser Phe Glu Gln Val Gly Thr Lys Val Ala Val Thr Val Glu
        115                 120                 125

Asp Glu Arg Thr Leu Val Arg Arg Asn Asn Thr Phe Leu Ser Leu Arg
    130                 135                 140

Asp Val Phe Gly Lys Asp Leu Ile Tyr Thr Leu Tyr Tyr Trp Lys Ser
145                 150                 155                 160

Ser Ser Ser Gly Lys Lys Thr Ala Lys Thr Asn Thr Asn Glu Phe Leu
                165                 170                 175

Ile Asp Val Asp Lys Gly Glu Asn Tyr Cys Phe Ser Val Gln Ala Val
            180                 185                 190

Ile Pro Ser Arg Thr Val Asn Arg Lys Ser Thr Asp Ser Pro Val Glu
        195                 200                 205

Cys Met Gly Gln Glu Lys Gly Glu Phe Arg Glu Ile Phe Tyr Ile Ile
    210                 215                 220

Gly Ala Val Val Phe Val Val Ile Ile Leu Val Ile Ile Leu Ala Ile
225                 230                 235                 240

Ser Leu His Lys Cys Arg Lys Ala Gly Val Gly Gln Ser Trp Lys Glu
                245                 250                 255

Asn Ser Pro Leu Asn Val Ser His His His His His His
            260                 265

<210>  6
<211>  813
<212>  DNA
<213>  artificial sequence

<220>
<223>  DNA encoding human mature TF with N124A and C-terminal

<400>  6
atgtcaggca ctacaaatac tgtggcagca tataatttaa cttggaaatc aactaatttc      60

aagacaattt tggagtggga acccaaaccc gtcaatcaag tctacactgt tcaaataagc     120

actaagtcag gagattggaa aagcaaatgc ttttacacaa cagacacaga gtgtgacctc     180

accgacgaga ttgtgaagga tgtgaagcag acgtacttgg cacgggtctt ctcctacccg     240

gcagggaatg tggagagcac cggttctgct ggggagcctc tgtatgagaa ctccccagag     300

ttcacacctt acctggagac aaacctcgga cagccaacaa ttcagagttt tgaacaggtg     360

ggaacaaaag tggcagtgac cgtagaagat gaacggactt tagtcagaag gaacaacact     420

ttcctaagcc tccgggatgt ttttggcaag gacttaattt atacacttta ttattggaaa     480

tcttcaagtt caggaaagaa aacagccaaa acaaacacta atgagttttt gattgatgtg     540

gataaaggag aaaactactg tttcagtgtt caagcagtga ttccctcccg aacagttaac     600

cggaagagta cagacagccc ggtagagtgt atgggccagg agaaagggga attcagagaa     660

atattctaca tcattggagc tgtggtattt gtggtcatca tccttgtcat catcctggct     720

atatctctac acaagtgtag aaaggcagga gtggggcaga gctggaagga gaactcccca     780

ctgaatgttt cacatcacca tcaccatcac tag                                  813

<210>  7
<211>  33
<212>  DNA
<213>  artificial sequence

<220>
<223>  upstream primer encoding the first amino acids of mature TF
       lacking the signal peptide and containing an initiation codon ATG
       in frame with TF ORF, and a restriction BamHI site

<400> 7
gggatccgca tgtcaggcac tacaaatact gtg 33

<210> 8
<211> 50
<212> DNA
<213> artificial sequence

<220>
<223> downstream primer containing the histidine coding nucleotides
and a new termination signal TAG, both containing a restriction
BamHI site

<400> 8
cggatccgtg tcgacctagt gatggtgatg gtgatgtgaa acattcagtg 50

<210> 9
<211> 33
<212> DNA
<213> artificial sequence

<220>
<223> primer

<400> 9
gtgggaacaa aagtggcagt gaccgtagaa gat 33

<210> 10
<211> 27
<212> DNA
<213> artificial sequence

<220>
<223> primer

<400> 10
atcttctacg gtcactgcca cttttgt 27

<210> 11
<211> 1374
<212> DNA
<213> Homo sapiens

<400> 11
gggggcgccg ccggcccttt atagcgcgcg gggcaccggc tccccaagac tgcgagctcc 60

ccgcaccccc tcgcactccc tctggccggc ccagggcgcc ttcagcccaa cctccccagc 120

cccacgggcg ccacggaacc cgctcgatct cgccgccaac tggtagacat ggagacccct 180

gcctggcccc gggtcccgcg ccccgagacc gccgtcgctc ggacgctcct gctcggctgg 240

gtcttcgccc aggtggccgg cgcttcaggc actacaaata ctgtggcagc atataattta 300

acttggaaat caactaattt caagacaatt ttggagtggg aacccaaacc cgtcaatcaa 360

gtctacactg ttcaaataag cactaagtca ggagattgga aaagcaaatg cttttacaca 420

acagacacag agtgtgacct caccgacgag attgtgaagg atgtgaagca gacgtacttg 480

gcacgggtct tctcctaccc ggcagggaat gtggagagca ccggttctgc tggggagcct 540

ctgtatgaga actccccaga gttcacacct tacctggaga caaacctcgg acagccaaca 600

attcagagtt ttgaacaggt gggaacaaaa gtgaatgtga ccgtagaaga tgaacggact 660

ttagtcagaa ggaacaacac tttcctaagc ctccgggatg tttttggcaa ggacttaatt 720

tatacacttt attattggaa atcttcaagt tcaggaaaga aaacagccaa aacaaacact 780

aatgagtttt tgattgatgt ggataaagga gaaaactact gtttcagtgt tcaagcagtg 840

attccctccc gaacagttaa ccggaagagt acagacagcc cggtagagtg tatgggccag 900

gagaaagggg aattcagaga aatattctac atcattggag ctgtggtatt tgtggtcatc 960

atccttgtca tcatcctggc tatatctcta cacaagtgta gaaaggcagg agtggggcag 1020

agctggaagg agaactcccc actgaatgtt tcataaagga agcactgttg gagctactgc 1080

aaatgctata ttgcactgtg accgagaact tttaagagga tagaatacat ggaaacgcaa 1140

atgagtattt cggagcatga agaccctgga gttcaaaaaa ctcttgatat gacctgttat 1200

taccattagc attctggttt tgacatcagc attagtcact ttgaaatgta acaaatggta 1260

ctacaaccaa ttccaagttt taatttttaa caccatggca ccttttgcac ataacatgct 1320

ttagattata tattccgccg tcccaaaaaa aaaaaaaaaa aaaaaaaaaa aaaa 1374